# EUROPEAN PATENT APPLICATION

(11) **EP 4 353 181 A2**
(43) Date of publication of application: **17.04.2024**
(21) Application number: 23202401.8
(22) Date of filing: 09.10.2023
(51) Int. Cl.: A61B 34/30, A61B 34/00, A61M 25/01

(54) **DRIVETRAIN FOR ELONGATED MEDICAL DEVICE**

(30) Priority: 12.10.2022 US 202218045842
(71) Applicant: Corindus, Inc., Newton, MA 02466 (US)
(72) Inventor: SAEEDI, Kody, Ashland, MA 01721 (US); BRITO, Arturo, Boston, MA 02116 (US); FALB, Peter, Hingham, MA 02043 (US); ZIRPS, Christopher, Sharon, MA 02067 (US)
(74) Representative: Horn Kleimann Waitzhofer Schmid-Dreyer Patent- und Rechtsanwälte PartG mbB

(57) **Abstract**

An EMD drive system in one implementation comprises a robotic drive having a robotic drive longitudinal axis; a device module movable along the robotic drive longitudinal axis, the device module including a motor having a motor shaft being substantially parallel to the robotic drive longitudinal axis; and a drive train coupling the motor shaft to a driven member configured to rotate an elongated medical device about an EMD longitudinal axis.

## Description

### FIELD

The present invention relates generally to the field of robotic medical procedure systems and, in particular, to a drivetrain for robotically controlling the movement and operation of elongated medical devices.

### BACKGROUND

Catheters and other elongated medical devices (EMDs) may be used for minimally invasive medical procedures for the diagnosis and treatment of diseases of various vascular systems, including neurovascular intervention (NVI) also known as neurointerventional surgery, percutaneous coronary intervention (PCI) and peripheral vascular intervention (PVI). These procedures typically involve navigating a guidewire through the vasculature, and via the guidewire advancing a catheter to deliver therapy. The catheterization procedure starts by gaining access into the appropriate vessel, such as an artery or vein, with an introducer sheath using standard percutaneous techniques. Through the introducer sheath, a sheath or guide catheter is then advanced over a diagnostic guidewire to a primary location such as an internal carotid artery for NVI, a coronary ostium for PCI, or a superficial femoral artery for PVI. A guidewire suitable for the vasculature is then navigated through the sheath or guide catheter to a target location in the vasculature. In certain situations, such as in tortuous anatomy, a support catheter or microcatheter is inserted over the guidewire to assist in navigating the guidewire. The physician or operator may use an imaging system (e.g., fluoroscope) to obtain a cine with a contrast injection and select a fixed frame for use as a roadmap to navigate the guidewire or catheter to the target location, for example, a lesion. Contrast-enhanced images are also obtained while the physician delivers the guidewire or catheter so that the physician can verify that the device is moving along the correct path to the target location. While observing the anatomy using fluoroscopy, the physician manipulates the proximal end of the guidewire or catheter to direct the distal tip into the appropriate vessels toward the lesion or target anatomical location and avoid advancing into side branches.

Robotic catheter-based procedure systems have been developed that may be used to aid a physician in performing catheterization procedures such as, for example, NVI, PCI and PVI. Examples of NVI procedures include coil embolization of aneurysms, liquid embolization of arteriovenous malformations and mechanical thrombectomy of large vessel occlusions in the setting of acute ischemic stroke. In an NVI procedure, the physician uses a robotic system to gain target lesion access by controlling the manipulation of a neurovascular guidewire and microcatheter to deliver the therapy to restore normal blood flow. Target access is enabled by the sheath or guide catheter but may also require an intermediate catheter for more distal territory or to provide adequate support for the microcatheter and guidewire. The distal tip of a guidewire is navigated into, or past, the lesion depending on the type of lesion and treatment. For treating aneurysms, the microcatheter is advanced into the lesion and the guidewire is removed and several embolization coils are deployed into the aneurysm through the microcatheter and used to block blood flow into the aneurysm. For treating arteriovenous malformations, a liquid embolic is injected into the malformation via a microcatheter. Mechanical thrombectomy to treat vessel occlusions can be achieved either through aspiration and/or use of a stent retriever. Depending on the location of the clot, aspiration is either done through an aspiration catheter, or through a microcatheter for smaller arteries. Once the aspiration catheter is at the lesion, negative pressure is applied to remove the clot through the catheter. Alternatively, the clot can be removed by deploying a stent retriever through the microcatheter. Once the clot has integrated into the stent retriever, the clot is retrieved by retracting the stent retriever and microcatheter (or intermediate catheter) into the guide catheter.

In PCI, the physician uses a robotic system to gain lesion access by manipulating a coronary guidewire to deliver the therapy and restore normal blood flow. The access is enabled by seating a guide catheter in a coronary ostium. The distal tip of the guidewire is navigated past the lesion and, for complex anatomies, a microcatheter may be used to provide adequate support for the guidewire. The blood flow is restored by delivering and deploying a stent or balloon at the lesion. The lesion may need preparation prior to stenting, by either delivering a balloon for pre-dilation of the lesion, or by performing atherectomy using, for example, a laser or rotational atherectomy catheter and a balloon over the guidewire. Diagnostic imaging and physiological measurements may be performed to determine appropriate therapy by using imaging catheters or fractional flow reserve (FFR) measurements.

In PVI, the physician uses a robotic system to deliver the therapy and restore blood flow with techniques similar to NVI. The distal tip of the guidewire is navigated past the lesion and a microcatheter may be used to provide adequate support for the guidewire for complex anatomies. The blood flow is restored by delivering and deploying a stent or balloon to the lesion. As with PCI, lesion preparation and diagnostic imaging may be used as well.

When support at the distal end of a catheter or guidewire is needed, for example, to navigate tortuous or calcified vasculature, to reach distal anatomical locations, or to cross hard lesions, an over-the-wire (OTW) catheter or coaxial system is used. An OTW catheter has a lumen for the guidewire that extends the full length of the catheter. This provides a relatively stable system because the guidewire is supported along the whole length. This system, however, has some disadvantages, including higher friction, and longer overall length compared to rapid-exchange catheters (see below). Typically to remove or exchange an OTW catheter while maintaining the position of the indwelling guidewire, the exposed length (outside of the patient) of guidewire must be longer than the OTW catheter. A 300 cm long guidewire is typically sufficient for this purpose and is often referred to as an exchange length guidewire. Due to the length of the guidewire, two operators are needed to remove or exchange an OTW catheter. This becomes even more challenging if a triple coaxial, known in the art as a tri-axial system, is used (quadruple coaxial catheters have also been known to be used). However, due to its stability, an OTW system is often used in NVI and PVI procedures. On the other hand, PCI procedures often use rapid exchange (or monorail) catheters. The guidewire lumen in a rapid exchange catheter runs only through a distal section of the catheter, called the monorail or rapid exchange (RX) section. With a RX system, the operator manipulates the interventional devices parallel to each other (as opposed to with an OTW system, in which the devices are manipulated in a serial configuration), and the exposed length of guidewire only needs to be slightly longer than the RX section of the catheter. A rapid exchange length guidewire is typically 180-200 cm long. Given the shorter length guidewire and monorail, RX catheters can be exchanged by a single operator. However, RX catheters are often inadequate when more distal support is needed.

### SUMMARY

An EMD drive system comprises a robotic drive having a robotic drive longitudinal axis; a device module movable along the robotic drive longitudinal axis, the device module including a motor having a motor shaft being substantially parallel to the robotic drive longitudinal axis; and a drive train coupling the motor shaft to a driven member configured to rotate an elongated medical device about an EMD longitudinal axis.

In one implementation the motor has a first length along a motor longitudinal axis less than a second longitudinal length of the device module taken along a longitudinal axis parallel to the robotic drive longitudinal axis.

In one implementation the drive train includes a drive gear rotating about an axis perpendicular to a motor longitudinal axis of the motor shaft, the drive gear removably engaged with the driven member.

In one implementation the EMD drive system includes a stage member extending from the robotic drive, the stage member moving the device module along the robotic drive longitudinal axis, the device module being extending solely from a first side of the stage member.

In one implementation the drive train includes a worm gear and a worm wheel.

The EMD drive system of claim 5, wherein the drive train includes an intermediate shaft being parallel to the motor shaft, the worm gear being rotated by the intermediate shaft and the worm gear being intermediate a proximal end of the motor and a distal end of the motor.

In one implementation the EMD drive system includes a collet removably received within the device module between a collet in-use position and a collet external position, the collet being manually adjustable in the collet in-use position between a first fixed position affixing an EMD thereto and a second unfixed position in which the EMD is not affixed thereto.

In one implementation t the drive train rotates the collet about a collet longitudinal axis, the drive train rotating the EMD when the EMD is fixed to the collet.

In one implementation the device module includes a drive module and a cassette removably coupled to the drive module, the drive module including a housing supporting the motor and the drive gear, the cassette including the driven member and removably receiving the collet and the EMD.

In one implementation the collet includes a first portion operatively connected to the drive train and a second portion movable by a user with respect to the first portion to fix and unfix the EMD to the Collet. In one further implementation the second portion is proximal the first portion when the collet is in an in-use position within a cassette.

In one implementation the drive train prevents back drive in response to a force applied to the collet to adjust the collet between the first fixed position and the second unfixed position.

In one implementation the drive train rotates the collet about a longitudinal axis of the collet, the drive train rotating the EMD when the EMD is fixed to the collet.

In one implementation the collet includes a gear operationally engaged with the driven member and a proximal portion being manipulated by a user when the collet is in the collet in-use position.

In another embodiment an EMD drive system comprises a robotic drive having a robotic drive longitudinal axis, the robotic drive having a housing including a bottom surface being closest to a patient in a robotic drive in-use position; a drive module movable along the robotic drive longitudinal axis, the drive module extending from the robotic drive with a bracket defining a drive plane extending along the robotic drive longitudinal axis and extending perpendicular to the bottom surface, the drive module being extending solely from one side of the drive plane, the drive module including a motor and a drive train operationally coupling the motor to an EMD within the drive module; and a sterile barrier removably attached to the bottom surface of the robotic drive on a second side of the drive plane opposite the one side of the drive plane.

In one implementation the drive module includes a motor having a drive shaft that has a longitudinal axis that is parallel with the drive plane.

In one implementation the EMD drive system includes a cassette being removably attached to the drive module, and a collet being removably received within the cassette between a collet external position and a collet in-use position, a portion of the collet being operatively connected to the drive train.

In one implementation the drive train includes a worm gear preventing back drive of the drive train when the collet is manually adjustable in the collet in-use position between a first fixed position affixing the EMD to the collet and a second unfixed position in which the EMD is not affixed to the collet.

In another embodiment an EMD drive system comprises a robotic drive having a robotic drive longitudinal axis; a device module movable along the robotic drive longitudinal axis, the device module including a motor having a motor shaft; and
a drive train coupling the motor shaft to a driven member configured to rotate an elongated medical device about an EMD longitudinal axis, the drive train including a worm gear preventing back drive of the drive train when a force is applied to the drive train from an EMD.

In one implementation a motor longitudinal axis of the motor shaft is parallel to the robotic drive longitudinal axis.

In one implementation the drive train includes an intermediate shaft extending parallel to the motor shaft, the worm gear being positioned on the intermediate shaft, the driven member rotating about an axis spaced from and perpendicular to the motor longitudinal axis and a longitudinal axis of the intermediate shaft; and wherein the worm gear is positioned on the intermediate shaft.

### BRIEF DESCRIPTION OF THE DRAWINGS

FIG 1 is a schematic view of an exemplary catheter procedure system.
FIG 2 is a schematic block diagram of an exemplary catheter procedure system.
FIG 3 is an exploded view of a cassette assembly and robotic drive and drive modules of a catheter procedure system.
FIG 4 is a left plan view of the robotic drive and distal most drive module.
FIG 5 is a front plan view of the robotic drive and distal most drive module.
FIG 6 is a perspective view of a right angle drive.
FIG 7 is an exploded view of the right angle drive of FIG 6.
FIG 8 is a perspective view of the right angle drive of FIG 6 with the worm gear housing removed.
FIG 9 is a right plan view of the right angle drive of FIG 6.
FIG 10 is a partial top plan view of the device module with a collet in an in-use position.
FIG 11 is a front plan view of the cassette with the collet in an in-use position.
FIG 11A is a front plan view of the cassette of FIG 11 with a cover in the closed position.
FIG 12 is a perspective view of the drive train.
FIG 13 is a partial exploded view of a collet.

### DETAILED DESCRIPTION OF THE EXAMPLE EMBODIMENTS

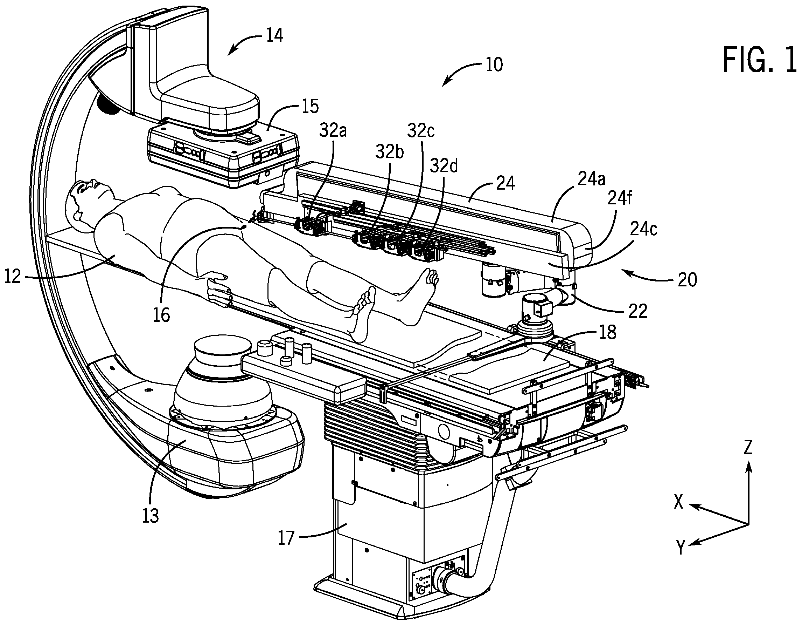

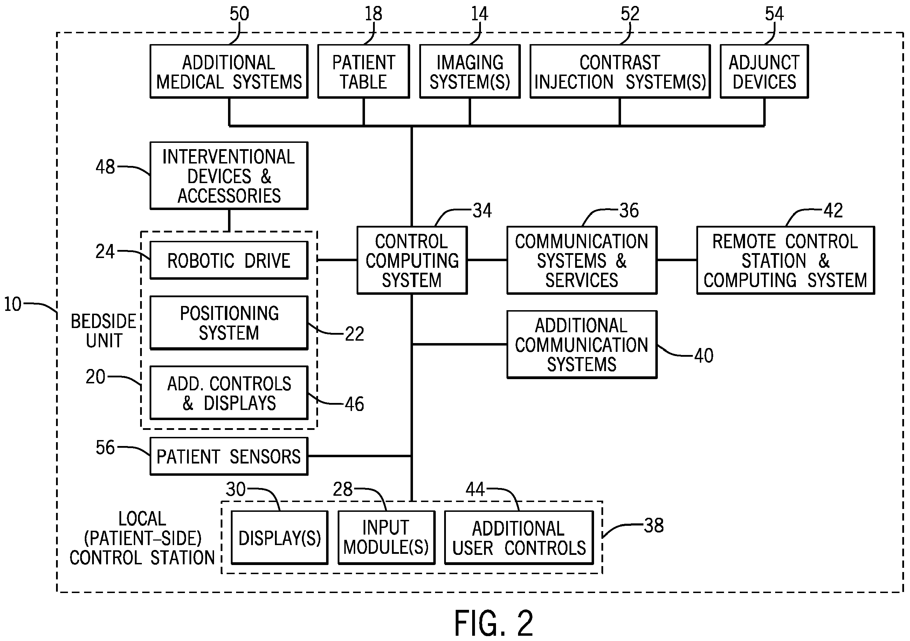

FIG. 1 is a perspective view of an example catheter-based procedure system 10 in accordance with an embodiment. Catheter-based procedure system 10 may be used to perform catheter-based medical procedures, e.g., percutaneous intervention procedures such as a percutaneous coronary intervention (PCI) (e.g., to treat STEMI), a neurovascular interventional procedure (NVI) (e.g., to treat an emergent large vessel occlusion (ELVO)), peripheral vascular intervention procedures (PVI) (e.g., for critical limb ischemia (CLI), etc.). Catheter-based medical procedures may include diagnostic catheterization procedures during which one or more catheters or other elongated medical devices (EMDs) are used to aid in the diagnosis of a patient's disease. For example, during one embodiment of a catheter-based diagnostic procedure, a contrast media is injected onto one or more arteries through a catheter and an image of the patient's vasculature is taken. Catheter-based medical procedures may also include catheter-based therapeutic procedures (e.g., angioplasty, stent placement, treatment of peripheral vascular disease, clot removal, arterial venous malformation therapy, treatment of aneurysm, etc.) during which a catheter (or other EMD) is used to treat a disease. Therapeutic procedures may be enhanced by the inclusion of adjunct devices 54 (shown in FIG. 2) such as, for example, intravascular ultrasound (IVUS), optical coherence tomography (OCT), fractional flow reserve (FFR), etc. It should be noted, however, that one skilled in the art would recognize that certain specific percutaneous intervention devices or components (e.g., type of guidewire, type of catheter, etc.) may be selected based on the type of procedure that is to be performed. Catheter-based procedure system 10 can perform any number of catheter-based medical procedures with minor adjustments to accommodate the specific percutaneous intervention devices to be used in the procedure.

Catheter-based procedure system 10 includes, among other elements, a bedside unit 20 and a control station (not shown). Bedside unit 20 includes a robotic drive 24 and a positioning system 22 that are located adjacent to a patient 12. Patient 12 is supported on a patient table 18. The positioning system 22 is used to position and support the robotic drive 24. The positioning system 22 may be, for example, a robotic arm, an articulated arm, a holder, etc. The positioning system 22 may be attached at one end to, for example, the patient table 18 (as shown in FIG. 1), a base, or a cart. The other end of the positioning system 22 is attached to the robotic drive 24. The positioning system 22 may be moved out of the way (along with the robotic drive 24) to allow for the patient 12 to be placed on the patient table 18. Once the patient 12 is positioned on the patient table 18, the positioning system 22 may be used to situate or position the robotic drive 24 relative to the patient 12 for the procedure. The position of the robotic drive in a position for the procedure is referred to herein as the robotic drive in-use position. In an embodiment, patient table 18 is operably supported by a pedestal 17, which is secured to the floor and/or earth. Patient table 18 is able to move with multiple degrees of freedom, for example, roll, pitch, and yaw, relative to the pedestal 17. Bedside unit 20 may also include controls and displays 46 (shown in FIG. 2). For example, controls and displays may be located on a housing of the robotic drive 24.

Generally, the robotic drive 24 may be equipped with the appropriate percutaneous interventional devices and accessories 48 (shown in FIG. 2) (e.g., guidewires, various types of catheters including but not limited to balloon catheters, stent delivery systems, stent retrievers, embolization coils, liquid embolics, aspiration pumps, device to deliver contrast media, medicine, hemostasis valve adapters, syringes, stopcocks, inflation device, etc.) to allow a user or operator to perform a catheter-based medical procedure via a robotic system by operating various controls such as the controls and inputs located at the control station. Bedside unit 20, and in particular robotic drive 24, may include any number and/or combination of components to provide bedside unit 20 with the functionality described herein. The robotic drive 24 includes a plurality of device modules 32a-d mounted to a rail or linear member. Each of the device modules 32a-d may be used to drive an EMD such as a catheter or guidewire. For example, the robotic drive 24 may be used to automatically feed a guidewire into a diagnostic catheter and into a guide catheter in an artery of the patient 12. One or more devices, such as an EMD, enter the body (e.g., a vessel) of the patient 12 at an insertion point 16 via, for example, an introducer sheath. Each device module 32a-d include a drive module and cassette removably attached to the drive module. Each drive module is movable along the robotic drive longitudinal axis with a bracket or stage. While FIG 1 illustrates four device modules it is contemplated that the number of device modules may be one or more.

Bedside unit 20 is in communication with the control station (not shown), allowing signals generated by the user inputs of the control station to be transmitted wirelessly or via hardwire to the bedside unit 20 to control various functions of bedside unit 20. As discussed below, control station 26 may include a control computing system 34 (shown in FIG. 2) or be coupled to the bedside unit 20 through the control computing system 34. Bedside unit 20 may also provide feedback signals (e.g., loads, speeds, operating conditions, warning signals, error codes, etc.) to the control station, control computing system 34 (shown in FIG. 2), or both. Communication between the control computing system 34 and various components of the catheter-based procedure system 10 may be provided via a communication link that may be a wireless connection, cable connections, or any other means capable of allowing communication to occur between components. The control station or other similar control system may be located either at a local site (e.g., local control station 38 shown in FIG. 2) or at a remote site (e.g., remote control station and computer system 42 shown in FIG. 2). Catheter procedure system 10 may be operated by a control station at the local site, a control station at a remote site, or both the local control station and the remote control station at the same time. At a local site, a user or operator and the control station are located in the same room or an adjacent room to the patient 12 and bedside unit 20. As used herein, a local site is the location of the bedside unit 20 and a patient 12 or subject (e.g., animal or cadaver) and the remote site is the location of a user or operator and a control station used to control the bedside unit 20 remotely. A control station (and a control computing system) at a remote site and the bedside unit 20 and/or a control computing system at a local site may be in communication using communication systems and services 36 (shown in FIG. 2), for example, through the Internet. In an embodiment, the remote site and the local (patient) site are away from one another, for example, in different rooms in the same building, different buildings in the same city, different cities, or other different locations where the remote site does not have physical access to the bedside unit 20 and/or patient 12 at the local site.

The control station generally includes one or more input modules 28 configured to receive user inputs to operate various components or systems of catheter-based procedure system 10. In the embodiment shown, control station allows the user or operator to control bedside unit 20 to perform a catheter-based medical procedure. For example, input modules 28 may be configured to cause bedside unit 20 to perform various tasks using percutaneous intervention devices (e.g., EMDs) interfaced with the robotic drive 24 (e.g., to advance, retract, or rotate a guidewire, advance, retract or rotate a catheter, inflate or deflate a balloon located on a catheter, position and/or deploy a stent, position and/or deploy a stent retriever, position and/or deploy a coil, inject contrast media into a catheter, inject liquid embolics into a catheter, inject medicine or saline into a catheter, aspirate on a catheter, or to perform any other function that may be performed as part of a catheter-based medical procedure). Robotic drive 24 includes various drive mechanisms to cause movement (e.g., axial and rotational movement) of the components of the bedside unit 20 including the percutaneous intervention devices.

In one embodiment, input modules 28 may include one or more touch screens, joysticks, scroll wheels, and/or buttons. In addition to input modules 28, the control station 26 may use additional user controls 44 (shown in FIG. 2) such as foot switches and microphones for voice commands, etc. Input modules 28 may be configured to advance, retract, or rotate various components and percutaneous intervention devices such as, for example, a guidewire, and one or more catheters or microcatheters. Buttons may include, for example, an emergency stop button, a multiplier button, device selection buttons and automated move buttons. When an emergency stop button is pushed, the power (e.g., electrical power) is shut off or removed to bedside unit 20. When in a speed control mode, a multiplier button acts to increase or decrease the speed at which the associated component is moved in response to a manipulation of input modules 28. When in a position control mode, a multiplier button changes the mapping between input distance and the output commanded distance. Device selection buttons allow the user or operator to select which of the percutaneous intervention devices loaded into the robotic drive 24 are controlled by input modules 28. Automated move buttons are used to enable algorithmic movements that the catheter-based procedure system 10 may perform on a percutaneous intervention device without direct command from the user or operator 11. In one embodiment, input modules 28 may include one or more controls or icons (not shown) displayed on a touch screen (that may or may not be part of a display), that, when activated, causes operation of a component of the catheter-based procedure system 10. Input modules 28 may also include a balloon or stent control that is configured to inflate or deflate a balloon and/or deploy a stent. Each of the input modules 28 may include one or more buttons, scroll wheels, joysticks, touch screen, etc. that may be used to control the particular component or components to which the control is dedicated. In addition, one or more touch screens may display one or more icons (not shown) related to various portions of input modules 28 or to various components of catheter-based procedure system 10.

Catheter-based procedure system 10 also includes an imaging system 14. Imaging system 14 may be any medical imaging system that may be used in conjunction with a catheter based medical procedure (e.g., non-digital X-ray, digital X-ray, CT, MRI, ultrasound, etc.). In an exemplary embodiment, imaging system 14 is a digital X-ray imaging device that is in communication with the control station. In one embodiment, imaging system 14 may include a C-arm (shown in FIG. 1) that allows imaging system 14 to partially or completely rotate around patient 12 in order to obtain images at different angular positions relative to patient 12 (e.g., sagittal views, caudal views, anterior-posterior views, etc.). In one embodiment imaging system 14 is a fluoroscopy system including a C-arm having an X-ray source 13 and a detector 15, also known as an image intensifier.

Imaging system 14 may be configured to take X-ray images of the appropriate area of patient 12 during a procedure. For example, imaging system 14 may be configured to take one or more X-ray images of the head to diagnose a neurovascular condition. Imaging system 14 may also be configured to take one or more X-ray images (e.g., real time images) during a catheter-based medical procedure to assist the user or operator 11 of control station 26 to properly position a guidewire, guide catheter, microcatheter, stent retriever, coil, stent, balloon, etc. during the procedure. The image or images may be displayed on display 30. For example, images may be displayed on a display to allow the user or operator to accurately move a guide catheter or guidewire into the proper position.

In order to clarify directions, a rectangular coordinate system is introduced with X, Y, and Z axes. The positive X axis is oriented in a longitudinal (axial) distal direction, that is, in the direction from the proximal end to the distal end, stated another way from the proximal to distal direction. The Y and Z axes are in a transverse plane to the X axis, with the positive Z axis oriented up, that is, in the direction opposite of gravity, and the Y axis is automatically determined by right-hand rule. As used herein the X axis extends along a longitudinal axis of the robotic drive 24. Since in an in-use position the robotic housing may be at an angle with respect to the horizontal plane perpendicular to the direction of gravity the X, Y and Z axes are defined by robotic drive 24. Referring to FIG 1, robotic drive 24 includes a housing having a top or first member 24a parallel to the X-Y plane; a bottom or second member 24b parallel to and spaced from the first surface 24a; a front or third member 24c substantially perpendicular and extending between first member 24a and second member 24b, the third member facing a user when robotic drive 24 is in the in-use position or orientation illustrated in FIG 1. A fourth member 24d is spaced from and substantially parallel to third member 24c and perpendicular to first member 24a and second member 24b. It is contemplated that other shapes of the robotic drive housing may be used. In which case first member 24a would be the upper member, second member 24b would be the lower or bottom member , front or third member 24c would be the portion facing a user in an in-use position during a surgical procedure, and fourth member 24d is the portion facing away from the user in the in-use position during a surgical procedure. Robotic drive 24 further includes a distal region 24e and a proximal region 24f. Where the distal region 24e is closer to the entry point of the patient through which the EMD will be introduced and the proximal region 24f is furthest from the entry point of the patient through which the EMD will be introduced.

FIG. 2 is a block diagram of catheter-based procedure system 10 in accordance with an example embodiment. Catheter-procedure system 10 may include a control computing system 34. Control computing system 34 may physically be, for example, part of a control station. Control computing system 34 may generally be an electronic control unit suitable to provide catheter-based procedure system 10 with the various functionalities described herein. For example, control computing system 34 may be an embedded system, a dedicated circuit, a general-purpose system programmed with the functionality described herein, etc. Control computing system 34 is in communication with bedside unit 20, communications systems and services 36 (e.g., Internet, firewalls, cloud services, session managers, a hospital network, etc.), a local control station 38, additional communications systems 40 (e.g., a telepresence system), a remote control station and computing system 42, and patient sensors 56 (e.g., electrocardiogram (ECG) devices, electroencephalogram (EEG) devices, blood pressure monitors, temperature monitors, heart rate monitors, respiratory monitors, etc.). The control computing system is also in communication with imaging system 14, patient table 18, additional medical systems 50, contrast injection systems 52 and adjunct devices 54 (e.g., IVUS, OCT, FFR, etc.). The bedside unit 20 includes a robotic drive 24, a positioning system 22 and may include additional controls and displays 46. As mentioned above, the additional controls and displays may be located on a housing of the robotic drive 24. Interventional devices and accessories 48 (e.g., guidewires, catheters, etc.) interface to the bedside unit 20. In an embodiment, interventional devices and accessories 48 may include specialized devices (e.g., IVUS catheter, OCT catheter, FFR wire, diagnostic catheter for contrast, etc.) which interface to their respective adjunct devices 54, namely, an IVUS system, an OCT system, and FFR system, etc.

In various embodiments, control computing system 34 is configured to generate control signals based on the user's interaction with input modules 28 (e.g., of a control station such as a local control station 38 or a remote control station 42) and/or based on information accessible to control computing system 34 such that a medical procedure may be performed using catheter-based procedure system 10. The local control station 38 includes one or more displays 30, one or more input modules 28, and additional user controls 44. The remote control station and computing system 42 may include similar components to the local control station 38. The remote 42 and local 38 control stations can be different and tailored based on their required functionalities. The additional user controls 44 may include, for example, one or more foot input controls. The foot input control may be configured to allow the user to select functions of the imaging system 14 such as turning on and off the X-ray and scrolling through different stored images. In another embodiment, a foot input device may be configured to allow the user to select which devices are mapped to scroll wheels included in input modules 28. Additional communication systems 40 (e.g., audio conference, video conference, telepresence, etc.) may be employed to help the operator interact with the patient, medical staff (e.g., angio-suite staff), and/or equipment in the vicinity of the bedside.

Catheter-based procedure system 10 may be connected or configured to include any other systems and/or devices not explicitly shown. For example, catheter-based procedure system 10 may include image processing engines, data storage and archive systems, automatic balloon and/or stent inflation systems, medicine injection systems, medicine tracking and/or logging systems, user logs, encryption systems, systems to restrict access or use of catheter-based procedure system 10, etc.

As mentioned, control computing system 34 is in communication with bedside unit 20 which includes a robotic drive 24, a positioning system 22 and may include additional controls and displays 46 and may provide control signals to the bedside unit 20 to control the operation of the motors and drive mechanisms used to drive the percutaneous intervention devices (e.g., guidewire, catheter, etc.). The various drive mechanisms may be provided as part of a robotic drive 24.
(Include description of linear movement of drive modules with linear member and bracket)

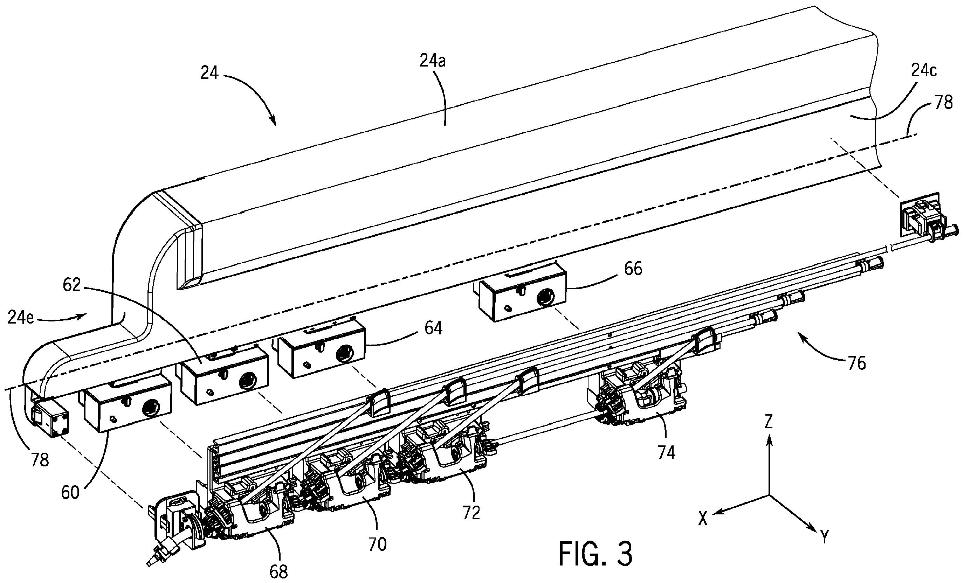

Referring to FIG 3 device module 32a includes a first drive module 60 and a fist cassette 68. Device module 32b includes a second drive module 62 and a second cassette 70. Device module 32c includes a third drive module 64 and a third cassette 72, Device module 32d includes a fourth drive module 66 and a fourth cassette 74. In one implementation first cassette 68, second cassette 70, third cassette 72 and fourth cassette 74 are shipped together as a multi-unit cassette assembly. In one implementation the multi-unit cassette assembly 76 allows for each of the cassettes to be removably connected to their respective drive modules while slidably connected together. In one implementation each of the multiple device modules 32a-d may be independently actuated to move linearly along a linear member within robotic drive 24. Each device module 32a-d may independently move relative to each other and the linear member in the robotic drive. The drive mechanism moves each device module along a longitudinal axis 78 of robotic drive 24, also referred to herein as the robotic drive longitudinal axis 78. Robotic drive longitudinal axis 78 may extend along the linear member such as a screw drive along which device modules move or may be defined along another axis that is parallel to the linear member along which the device modules move. Referring to FIG 3 each cassette 68-74 are generally vertically oriented in the XZ plane. Each cassette 68-74 has a length along the X axis or parallel to longitudinal axis 78 that is greater than the width of each cassette along the Y axis or perpendicular to the Y axis. The `533 publication discloses a cassette positioned in a generally horizontal position along in the XY. The distinction between the vertical and horizontal of the cassettes is described in PCT International Publication No. WO 2021/011554 and incorporated herein by reference in its entirety.

In one implementation the drive mechanism includes independent stage translation motors coupled to each device module and a stage drive mechanism such as a lead screw via a rotating nut, a rack via a pinion, a belt via a pinion or pulley, a chain via a sprocket, or the stage translation motors 64a-d may be linear motors themselves. The drive mechanism provides for advancement and retraction of the device modules. Examples of such drive mechanisms are described in PCT International Publication No. WO 2021/011533 incorporated herein by reference in its entirety.

To prevent contaminating the patient with pathogens, healthcare staff use aseptic technique in a room housing the bedside unit 20 and the patient 12 or subject (shown in FIG. 1). A room housing the bedside unit 20 and patient 12 may be, for example, a cath lab or an angio suite. Aseptic technique consists of using sterile barriers, sterile equipment, proper patient preparation, environmental controls and contact guidelines. Accordingly, all EMDs and interventional accessories are sterilized and can only be in contact with either sterile barriers or sterile equipment. In an embodiment, a sterile drape (not shown) is placed over the non-sterile robotic drive 24. Each cassette 68-74 is sterilized and acts as a sterile interface between the draped robotic drive 24 and at least one EMD. Each cassette 68-74 can be designed to be sterile for single use or to be re-sterilized in whole or part so that the cassette 68-74 or its components can be used in multiple procedures.

Distal and Proximal: The terms distal and proximal define relative locations of two different features. With respect to a robotic drive the terms distal and proximal are defined by the position of the robotic drive in its intended use relative to a patient.
When used to define a relative position, the distal feature is the feature of the robotic drive that is closer to the patient than a proximal feature when the robotic drive is in its intended in-use position. Within a patient, any vasculature landmark further away along the path from the access point is considered more distal than a landmark closer to the access point, where the access point is the point at which the EMD enters the patient. Similarly, the proximal feature is the feature that is farther from the patient than the distal feature when the robotic drive in its intended in-use position. When used to define direction, the distal direction refers to a path on which something is moving or is aimed to move or along which something is pointing or facing from a proximal feature toward a distal feature and/or patient when the robotic drive is in its intended in-use position. The proximal direction is the opposite direction of the distal direction. By way of examples referring to FIG 1, a robotic device is shown from the viewpoint of an operator facing a patient. In this arrangement, the distal direction is along the positive X coordinate axis and the proximal direction is along the negative X coordinate axis.

Longitudinal axis: The term longitudinal axis of a member (for example, an EMD or other element in the catheter-based procedure system) is the line or axis along the length of the member that passes through the center of the transverse cross section of the member in the direction from a proximal portion of the member to a distal portion of the member. For example, the longitudinal axis of a guidewire is the central axis in the direction from a proximal portion of the guidewire toward a distal portion of the guidewire even though the guidewire may be non-linear in the relevant portion.

Axial Movement: The term axial movement of a member refers to translation of the member along the longitudinal axis of the member. When the distal end of an EMD is axially moved in a distal direction along its longitudinal axis into or further into the patient, the EMD is being advanced. When the distal end of an EMD is axially moved in a proximal direction along its longitudinal axis out of or further out of the patient, the EMD is being withdrawn.

Rotational Movement: The term rotational movement of a member refers to the change in angular orientation of the member about the local longitudinal axis of the member. Rotational movement of an EMD corresponds to clockwise or counterclockwise rotation of the EMD about its longitudinal axis due to an applied torque.

Axial and Lateral Insertion: The term axial insertion refers to inserting a first member into a second member along the longitudinal axis of the second member. An EMD that is axially loaded in a collet is axially inserted in the collet. An example of axial insertion could be referred to as back loading a catheter on the proximal end of a guidewire. The term lateral insertion refers to inserting a first member into a second member along a direction in a plane perpendicular to the longitudinal axis of the second member. This can also be referred to as radial loading or side loading. Stated another way, lateral insertion refers to inserting a first member into a second member along a direction that is parallel to the radius and perpendicular to the longitudinal axis of the second member.

Up/Down; Front/Rear; Inwardly/Outwardly: The terms top, up, and upper refer to the general direction away from the direction of gravity and the terms bottom, down, and lower refer to the general direction in the direction of gravity. The term front refers to the side of the robotic drive that faces a bedside user and away from the positioning system, such as the articulating arm. The term rear refers to the side of the robotic drive that is closest to the positioning system, such as the articulating arm. The term inwardly refers to the inner portion of a feature. The term outwardly refers to the outer portion of a feature.

Stage: The term stage refers to a member, feature, or device that is used to couple a device module to the robotic drive. For example, the stage may be used to couple the device module to a rail or linear member of the robotic drive.

Drive Module: The term drive module generally refers to the part (e.g., the capital part) of the robotic drive system that normally contains one or more motors with drive couplers that interface with the cassette.

Device Module: The term device module refers to the combination of a drive module and a cassette.

Cassette: The term cassette generally refers to the part (non-capital, consumable or sterilizable unit) of the robotic drive system that normally is the sterile interface between a drive module and at least one EMD (directly) or through a device adapter (indirectly).

Shaft (Distal) Driving: The term shaft (distal) driving refers to holding on to and manipulating an EMD along its shaft. In one example the on-device adapter is normally placed just proximal of the hub or Y-connector the device is inserted into. If the location of the on-device adapter is at the proximity of an insertion point (to the body or another catheter or valve), shaft driving does not typically require anti-buckling features. (It may include anti-buckling features to improve drive capability.)

Collet: The term collet refers to a device that can releasably fix a portion of an EMD. The term fixed here means no intentional relative movement of the collet and EMD during operation. In one embodiment the collet includes at least two members that move rotationally relative to each other to releasably fix the EMD to at least one of the two members. In one embodiment the collet includes at least two members that move axially (along a longitudinal axis) relative to each other to releasably fix the EMD to at least one of the two members. In one embodiment the collet includes at least two members that move rotationally and axially relative to each other to releasably fix the EMD to at least one of the two members.

Fixed: The term fixed means no intentional relative movement of a first member with respect to a second member during operation.

Pinch/Unpinch: The term pinch refers to releasably fixing an EMD to a member such that the EMD and member move together when the member moves. The term unpinch refers to releasing the EMD from a member such that the EMD is no longer fixed to a member but unfixed to that member and the EMD moves independently of the member.

On-Device Adapter: The term on-device adapter refers to a sterile apparatus capable of releasably pinching an EMD to provide a driving interface. The on-device adapter is also known as an end-effector or EMD capturing device. In one non-limiting embodiment the on-device adapter is a collet that is operatively controlled robotically to rotate the EMD about its longitudinal axis, to pinch and/or unpinch the EMD to the collet, and/or to translate the EMD along its longitudinal axis. In one embodiment the on-device adapter is a hub-drive mechanism such as a gear located on the hub of an EMD.

EMD: The term elongated medical device (EMD) refers to, but is not limited to, catheters (e.g., guide catheters, microcatheters, balloon/stent catheters), wire-based devices (e.g., guidewires, embolization coils, stent retrievers, etc.), and medical devices comprising any combination of these. In one example a wire-based EMD includes but is not limited to guidewires, microwires, a proximal pusher for embolization coils, stent retrievers, self-expanding stents, and flow divertors. Typically wire-based EMD's do not have a hub or handle at its proximal terminal end. In one embodiment the EMD is a catheter having a hub at a proximal end of the catheter and a flexible shaft extending from the hub toward the distal end of the catheter, wherein the shaft is more flexible than the hub. In one embodiment the catheter includes an intermediary portion that transitions between the hub and the shaft that has an intermediate flexibility that is less rigid than the hub and more rigid than the shaft. In one embodiment the intermediary portion is a strain relief.

Hub (Proximal) Driving: The term hub driving, or proximal driving refers to holding on to and manipulating an EMD from a proximal position (e.g., a geared adapter on a catheter hub). In one embodiment, hub driving refers to imparting a force or torque to the hub of a catheter to translate and/or rotate the catheter. Hub driving may cause the EMD to buckle and thus hub driving often requires anti-buckling features. For devices that do not have hubs or other interfaces (e.g., a guidewire), device adapters may be added to the device to act as an interface for the device module. In one embodiment, an EMD does not include any mechanism to manipulate features within the catheter such as wires that extend from the handle to the distal end of the catheter to deflect the distal end of the catheter.

Sterilizable Unit: The term sterilizable unit refers to an apparatus that is capable of being sterilized (free from pathogenic microorganisms). This includes, but is not limited to, a cassette, consumable unit, drape, device adapter, and sterilizable drive modules/units (which may include electromechanical components). Sterilizable Units may come into contact with the patient, other sterile devices, or anything else placed within the sterile field of a medical procedure.

Sterile Interface: The term sterile interface refers to an interface or boundary between a sterile and non-sterile unit. For example, a cassette may be a sterile interface between the robotic drive and at least one EMD.

Consumable: The term consumable refers to a sterilizable unit that normally has a single use in a medical procedure. The unit could be a reusable consumable through a re-sterilization process for use in another medical procedure.

Gear: The term gear may be a bevel gear, spiral bevel gear, spur gear, miter gear, worm gear, helical gear, rack and pinon, screw gear, internal gear such as a sun gear, involute spline shafts and bushing, or any other type of gears known in the art.

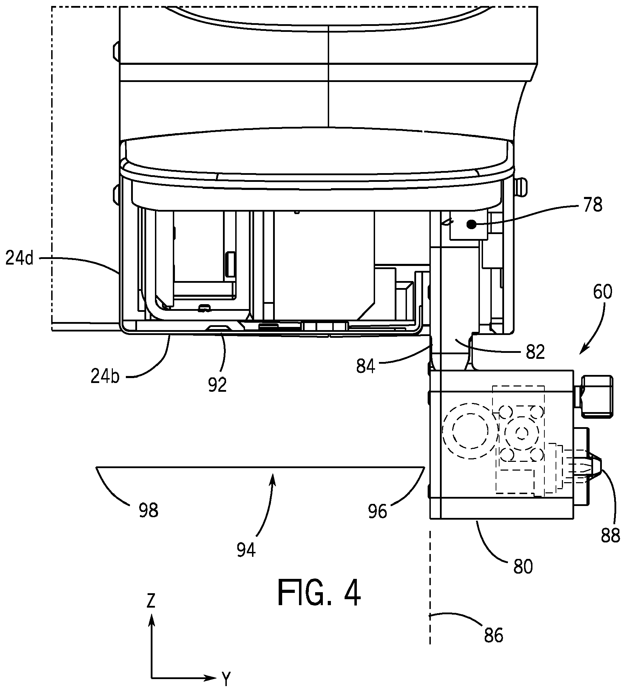

Referring to FIG 4, first drive module 60 will serve as an exemplary drive module. One or all of second drive module 62, third drive module 64 and fourth drive module 66 may be identical to first drive module. First drive module 60 includes a housing 80 that is operatively connected to robotic drive 24 with a stage member or bracket 82. A bracket plane 86 defined by the Z axis and X axis is located on the side 84 of bracket 82. Side 84 is spaced from the drive member 88 that engages with a driven member 90 of cassette 68. Housing 80 extends solely frontward from side 84 of bracket 82. Stated another way, side 84 of bracket 82 is closely adjacent or rearward (along the -Y axis) from housing 80. Drive member 88 is often referred to as a capstan and will be used interchangeably herein. Capstan 88 is the output gear of actuator 100 that performs the function as a drive member in the sense that capstan 88 is the member that drives driven member 90 in cassette 68. The term drive member, capstan and output gear are used interchangeably herein. In one implementation housing 80 is located solely on a first side of the bracket plane 86 that is closest to the driven member 90. Robotic drive 24 includes a housing including a bottom surface 92 having a longitudinal opening through which bracket 82 extends. Drive module 60 is located below the bottom surface 92 of the robotic drive housing. A sterile barrier 94 is removably attached to robotic drive 24 covering the bottom surface 92 on the side of the bracket plane 86 distal to the output gear 88. In one implementation sterile barrier 94 may be raised from a first lower position to the bottom surface 92 in the Z axis direction immediately adjacent bracket 82. In one implementation sterile barrier 94 includes a first longitudinal edge 96 proximate the bracket plane 86 when sterile barrier 94 is operatively secured to bottom surface 92 of robotic drive 24. Referring to FIG 4 in one implementation sterile barrier 94 includes a second longitudinal edge 98 that is secured to a portion of bottom surface 92. In one implementation sterile barrier 94 covers bottom surface 92 and second longitudinal edge 98extends about bottom surface 92 and is secured to a fourth member 24d of robotic drive housing. Sterile barrier 94 may be a formed from a flexible material or a rigid material. In one implementation sterile barrier 94 includes a planar portion that is moved into position against bottom surface 92 in a direction perpendicular to a plane defined by the bottom surface. Referring to FIG 4 sterile barrier 94 would move in a direction parallel to the Z axis and the bottom surface 92 would be parallel to a plane defined by the X axis and Y axis. In one implementation each drive module extends below bottom surface 92. Stated another way when robotic drive is in an in-use position each respective motor within motor drive module is located in a plane defined by the bottom surface 92 and the patient.

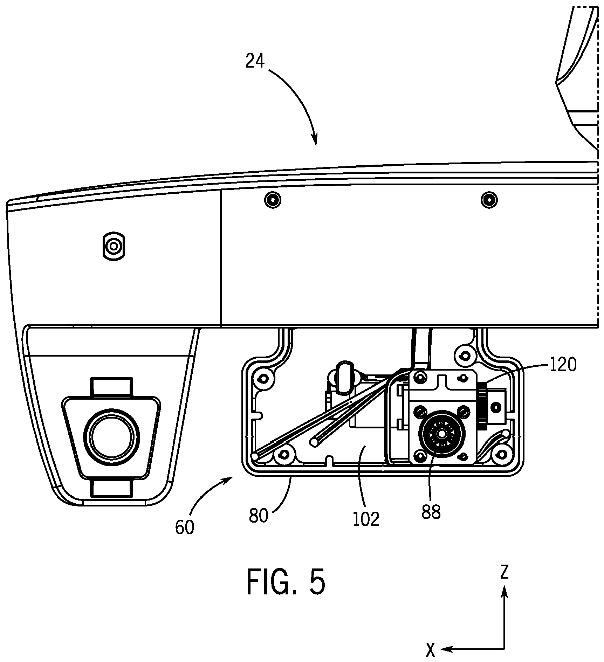

Referring to FIG 5,FIG 6 and FIG 7, motor 102 has a first length along motor longitudinal axis 114 that is less than a second longitudinal length of the device module housing 80 taken along a longitudinal axis parallel to the robotic drive longitudinal axis 78. Referring to FIG 5, the lengths of the motor and housing 80 is taken along the X-axis. In one implementation the combined length of motor encoder 106, motor housing 112, motor shaft 108, motor drive shaft gear 110 and gear hub 144 fits fully within housing 80 of drive module 60. In one implementation the combined length of motor 102, motor housing 112, motor drive shaft 108, motor drive shaft gear 110 and gear hub 144 are located within the portion of drive module 60 housing below bottom surface 92 of the robotic drive housing.

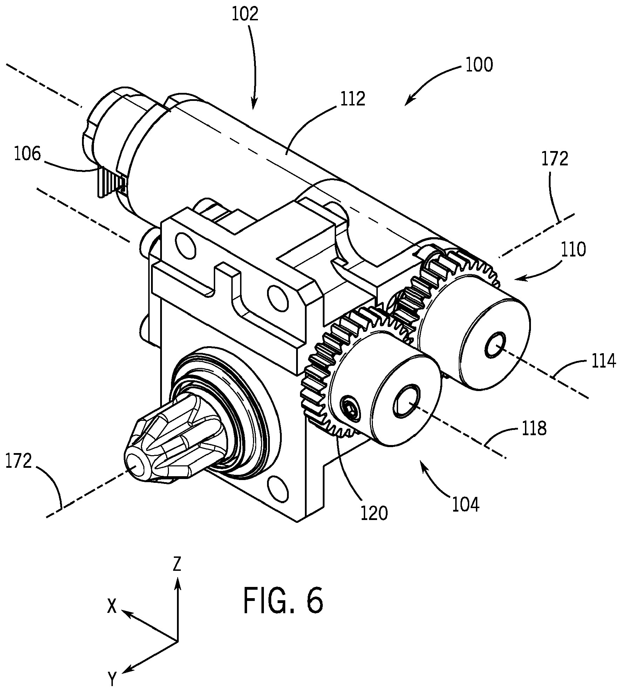

Referring to FIG 6, FIG 7 and FIG 8 an actuator assembly 100 includes a motor 102 and a drive train 104. As discussed herein, drive train 104 operationally couples the motor 102 to an EMD within the drive module. In one implementation drive train 104 changes the speed, torque, and/or direction of the motor output. The change of direction of the output of drive train 104 may be parallel to, perpendicular to, or otherwise not co-linear with the motor output drive shaft 108. Motor 102 includes an encoder 106 receiving instructions from control station 26 to operate motor 102. In one implementation motor 102 is a servomotor that receives a control signal via encoder 106 that applies power to the motor until the motor drive shaft 108 turns to an exact position determined by a position sensor. Motor 102 includes a motor drive shaft 108. Motor 102 includes a motor housing 112 through which motor drive shaft 108 extends. A motor drive shaft gear 110 is operatively coupled to motor drive shaft 108 closely adjacent to the proximal end of motor housing 112. In one implementation, motor drive shaft gear 110 is a spur gear. In one implementation motor 102, motor housing 112 and motor drive shaft 108 have a longitudinal axis 114 that is parallel to longitudinal axis 78 of robotic drive 24. Drive train 104 further includes a second shaft 116 that has a longitudinal axis 118 that is parallel to motor drive shaft 108 of motor 102. The proximal end of second shaft 116 is adjacent to the proximal end of motor drive shaft 108. A second gear 120 is secured to the proximal end of second shaft 116. Second gear 120 interacts with motor drive shaft gear 110. In one implementation motor drive shaft gear 110 and second gear 120 are spur gears.

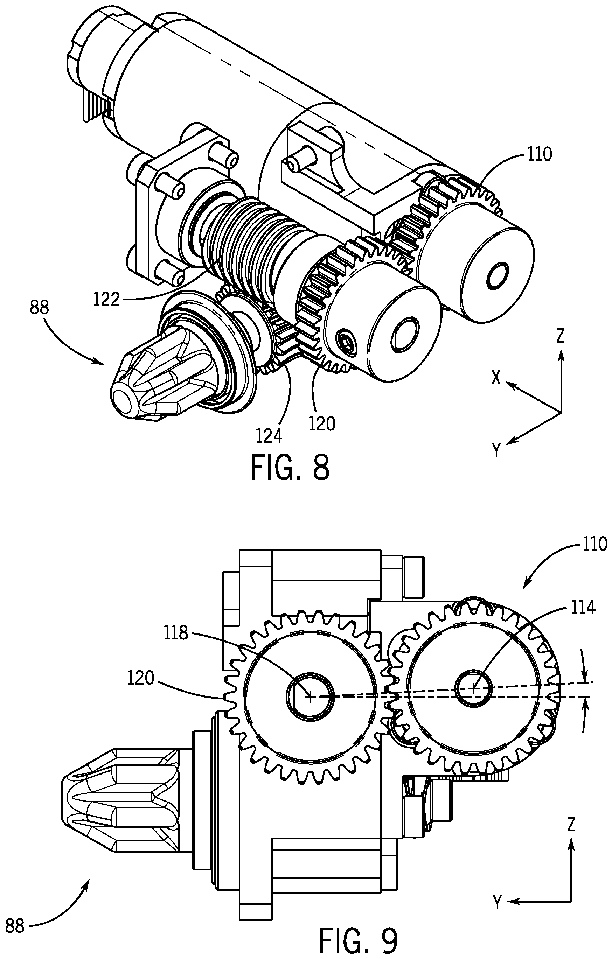

Referring to FIG 9 longitudinal axis 118 of second shaft 116 is offset from longitudinal axis 114 such that a line between longitudinal axis 114 and longitudinal axis 118 is approximately three degrees from Y axis. However other angle offsets are also contemplated. In one implementation the angle offset is between one degree and ten degrees. The angle offset for greater clearance between the outer circumference of motor drive shaft gear 110 and housing 80 of drive module 60.

A worm gear 122 is secured to or part of second shaft 116 and drives a worm wheel 124. Worm wheel 124 rotates about an axis that is offset from and perpendicular to longitudinal axis 118. Worm wheel 124 is secured to a worm wheel shaft 126 that extends in a direction parallel to the Y axis. Output gear 88 is secured to worm wheel shaft 126. In one implementation worm wheel shaft 126 is secured to or integral with output gear 88. In one implementation output gear 88 is a capstan or geared member that is received in a driven member 90 having a cavity that receives and/or meshes with the teeth of output gear 88. In one implementation driven member 90 includes a gear portion 128 that interacts with a gear 174 on the on-device adapter 142. In one implementation output gear 88 is a gear that drives a driven member 90 in cassette 68. However driven member 90 may be directly secured to an on-device adapter 142 that releasably fixes an elongated EMD thereto.

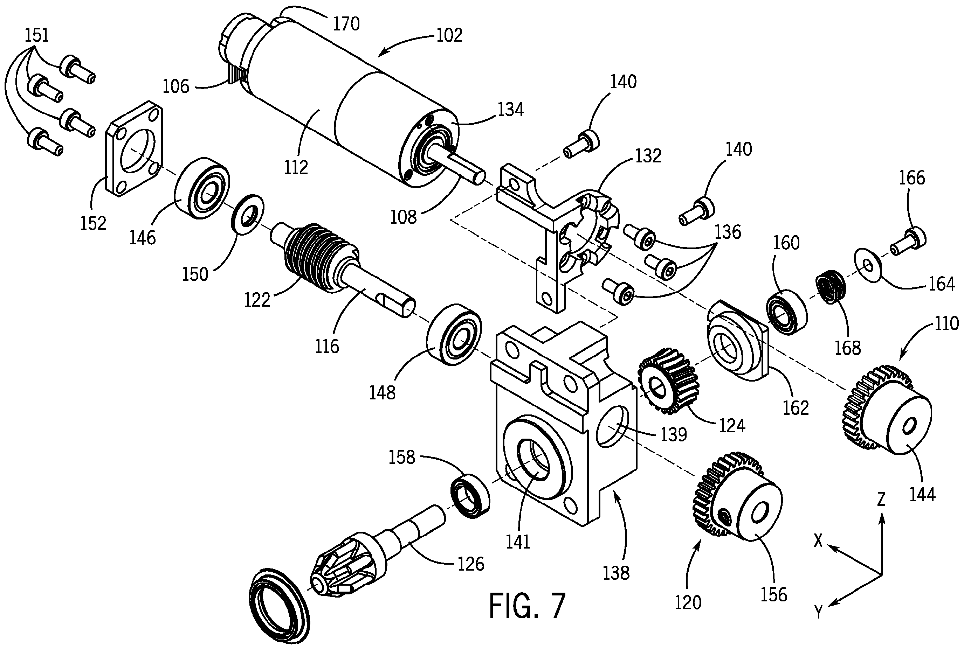

Referring to FIG 7 a motor bracket 132 is connected to a proximal end 134 of motor 102 with a plurality of fasteners 136. Note that the terms proximal and distal as used herein refer to the orientation of the features being discussed when the feature in an in-use position in robotic drive 24. Motor bracket 132 is secured to a worm gear housing 138 with fasteners 140. Motor drive shaft gear 110 is connected to proximal end of motor drive shaft 108. In one implementation motor drive shaft 108 is keyed and fits into a corresponding keyed opening in the body of the motor drive shaft gear 110. Worm gear housing 138 includes a first opening 139 receiving second shaft 116 and a second opening 141 receiving the worm wheel 124. Motor drive shaft gear 110 includes a gear hub 144 that received the proximal portion of motor drive shaft 108. In one implementation a set screw secures gear hub 144 to the proximal end of motor drive shaft 108. Second shaft 116 freely rotates within worm gear housing 138 about a first bearing 146 and a second bearing 148. In one implementation a washer 150 is positioned intermediate first bearing 146 and worm gear 122. In one implementation washer 150 is a Belleville disc spring. In other implementations washer 150 can be any kind of spring that provides a preloaded force to the worm shaft. An end cap 152 is attached to worm gear housing 138 with a plurality of fasteners 151. Second shaft 116 is rotatingly retained within worm gear housing 138 between end cap 152 and a gear hub 156 of second gear 120. Gear hub 156 is secured to a proximal portion of second shaft 116 with a set screw or other known methods of securing a gear housing to a shaft that is known in the art including but not limited to a friction fit.

Worm wheel shaft 126 is rotatingly secured to worm gear housing 138 with support of a first bearing 158 and a second bearing 160. A bearing holder 162 positions second bearing 160 relative to worm gear housing 138. A bearing cap 164 is secured to worm wheel shaft 126 with a fastener 166. A spring 168 is used to provide a preload to the capstan shaft 126 which is attached to worm wheel 124. Spring 168 provides a preload to bearing 158 and bearing 160. In one implementation spring 168 also functions as a disc spring washer.

Worm gear 122 is positioned on worm wheel shaft 126 on longitudinal axis 118 that is offset from motor longitudinal axis 114. Worm gear 122 is positioned offset from motor housing 112 and between proximal end proximal end 134 and a distal end 170 of motor housing 112. Worm wheel shaft 126 and output gear 88 is offset from longitudinal axis 118 of motor 102 and is between proximal end 134 and distal end 170 of motor housing 112. In one implementation the entire motor, worm gear and worm wheel are entirely contained within the housing of the drive module. In one implementation the motor is positioned within the drive module 60 along a longitudinal axis 114 that is offset from and perpendicular to the direction of the drive gear longitudinal axis 172. In one implementation the longitudinal axis 114 of motor 102 is located within a plane that is within or parallel to bracket plane 86. In one implementation the bottom surface 92 of housing 80 defines a bottom surface plane that is co-planar with or parallel to or a plane defined by the X axis and Y axis as illustrated in the Figures. Bracket plane 86 is perpendicular to by the bottom surface plane. Co-longitudinal axis 114 of motor 102 is located within a plane or parallel to bracket plane 86. Output gear 88 rotates about worm wheel shaft 126 that is perpendicular to bracket plane 86.

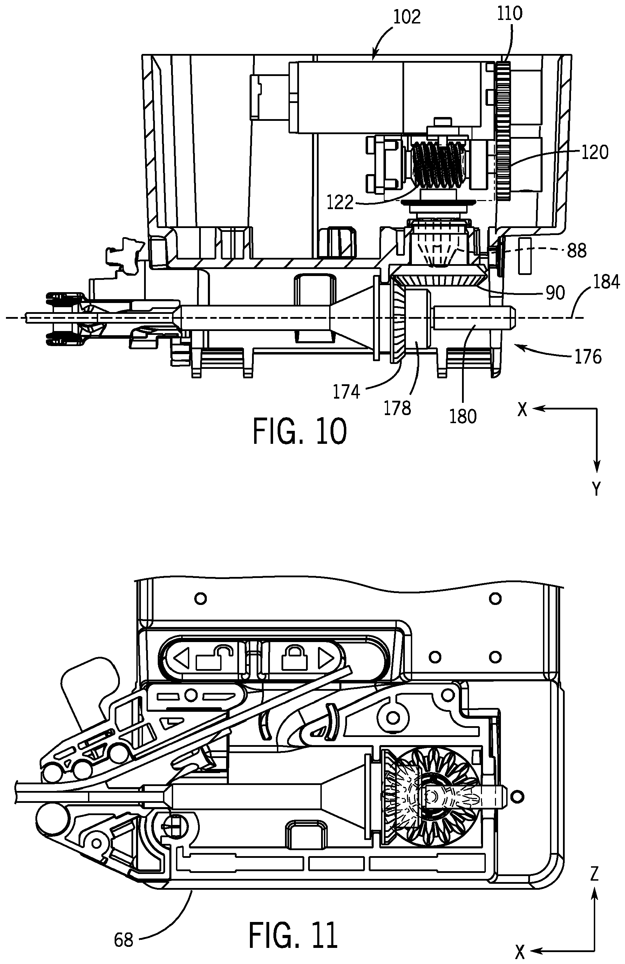

Referring to FIGS 10-12 an on-device adapter 142 includes a gear 174 that is rotated by the driven gear 90. In one implementation gear 174 is a beveled gear that is rotated by driven member 90. In one implementation gear 174 is a driven gear that is driven directly by output gear 88. In one implementation on-device adapter 142 includes a collet 176 having a first portion 178 and a second portion 180 that may be manually rotated about a longitudinal axis 184 that extends longitudinally through collet 176, on-device adapter 142. In one implementation a guide wire 182 or other EMD is releasably fixed to collet 176. Stated another way, collet 176 has a position in which the EMD is fixed to the collet and a second unfixed position in which the EMD is not fixed to the collet. In one implementation first portion 178 of collet 176 is operatively connected to actuator 100. Second portion 180 of collet 176 is movable relative to first portion 178 of collet 176. In one implementation first portion 178 is rotated about the longitudinal axis of collet 176 in a first direction to affix guide wire 182 to collet 176 such that rotation of first portion 178 by actuator assembly 100 also rotates guide wire 182. Further movement of device module 32a along the robotic drive longitudinal axis 78 will move guide wire 182 along an EMD longitudinal axis which is parallel to and offset from longitudinal axis 78 or robotic drive 24. In one implementation second portion 180 of collet 176 extends in a proximal direction from the first portion 178 of collet 176. In one implementation a proximal end of second portion 180 of collet 176 extends in a proximal direction from second portion 180. In one implementation the proximal end of portion 180 of collet 176 is freely grip-able by a user and free from any cassette housing structure that would impede a user from grasping and manipulating 180 when collet 176 is an in-use position in cassette 68. In operation, collet 176 is placed from an external position outside of the cassette to an in-use position in which the collet that rotated relative to cassette and move linearly along longitudinal axis 184. Stated another way the in-use position of the collet is in when the robotic drive rotates and translates the EMD as generally described herein.

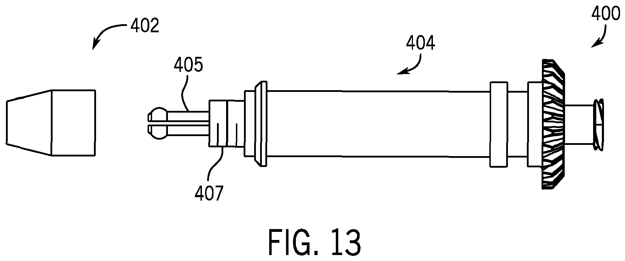

The on-device adapter and collet 176 is shown schematically in FIGS 10 - 12. Examples of an on-device adapter and various collet designs are disclosed in PCT International Publication No. WO 2021/011533 (the `533 Publication) incorporated herein by reference in its entirety hereinabove. Referring to FIG 13 a collet 400 disclosed in the `533 Publication and shown in FIG 6A of the `533 Publication a first member 402 (nut) moving along and/or about a longitudinal axis 406 of the second member 404 (collet body) to pinch the shaft of an elongated medical device such as a guide wire within a third member 405 (chuck). Nut 402 is threadedly engaged with collet body 404 at a threaded portion 407. Nut 402 is manually threaded on collet body 404 to pinch and unpinch the shaft of the elongated medical device. Collet 400 provides one example a collet and its operation. While the general operation of collet 176 in one implementation is similar to the operation of collet 400, the second portion 180 of collet 176 as described herein that is manually rotated is positioned proximal to the first portion 178 when collet 176 in an in-use position within cassette 68. Gear 174 secured to first portion 178 is distal to second portion 180 when collet 176 is in the in-use position within cassette 68. Other types of collets disclosed in the `533 Publication, known in the art or invented hereinafter may be used in connection with the embodiments and implementations disclosed herein. In one implementation a cover 188 is pivotally attached to cassette 68 from an open position to a closed in-use position. Second portion 180 of collet 176 is accessible for manual manipulation by a user when cover 188 is in the closed in-use position.

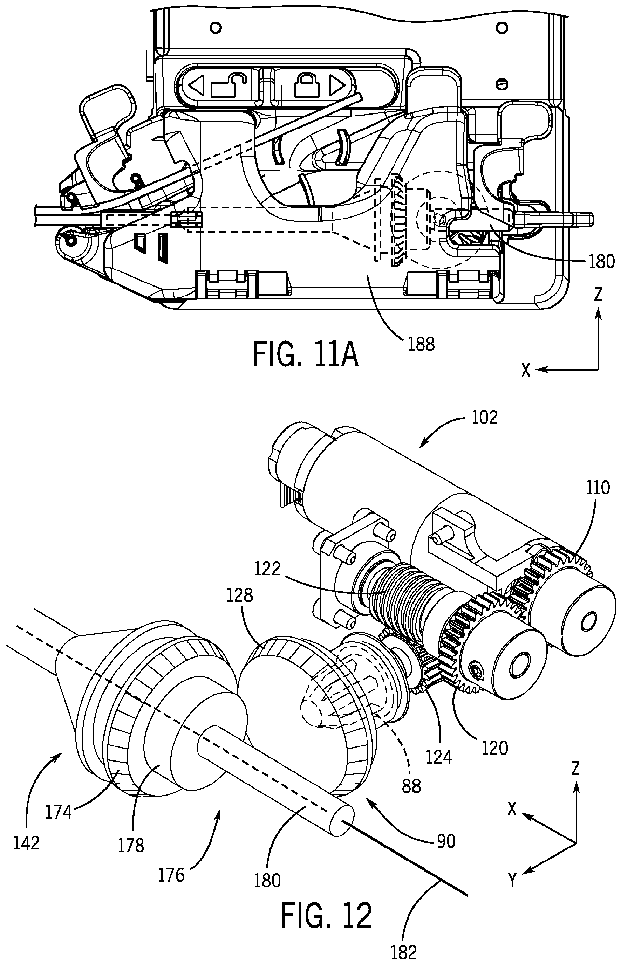

Referring to FIG 11A there is sufficient clearance in cassette 68 to permit a user to grip second portion 180 of collet 176 and rotate second portion 180 about longitudinal axis 184 thereby affixing or releasing guide wire 182 thereto or therefrom. The torque applied by a user to second portion 180 will provide a torque to the drive train 104 that could result in the back driving of drive train 104. The worm gear prevents back driving of the drive train 104 and acts as a brake in both directions of rotation against a Torque applied by a user to collet 176. Back driving of the drive train 104 entails movement of the features such as gears within drive train 104 without instruction from encoder 106. Worm gear 122 within drive train 104 prevents back driving of the gears within drive train 104. In one implementation drive train 104 provides back driving against a manual torque applied by a user to collet 176 in the collet in-use position in cassette as EMD 182 is being fixed and unfixed to collet 176. In one implementation device module does not include a separate brake mechanism to prevent drive train 104 from back driving upon the application of a manual force to either fix or unfix an EMD to the collet as the worm gear 122 prevents back driving of the force. In one implementation a separate brake member is provided to lock drive train 104 from moving during the manual application of a torque to either fix or unfix an EMD to the collet. In one implementation a clutch (not shown) is positioned between the drivetrain 104 and collet 176 such that a user may be able to choose whether they want to prevent back driving.

Collet 176 is a device that releasably fixes a shaft portion of EMD 182 thereto. As described in more detail herein collet 176 pinches the shaft of EMD 182 such that rotation and/or translation of the entire collet 176 about or along its longitudinal axis 184 results in the same rotation and/or translation of the portion of the shaft of EMD 182 that is pinched. In one embodiment collet 176 may be a single molded component having a body defining an internal pathway through which a portion of the shaft of the EMD 182 may be fixed. As described herein the shaft of the EMD 182 is positioned in the internal pathway of the collet and pinched therein. The shaft of the EMD 182 may be radially loaded or axially-loaded into the internal pathway of the collet. Radially loaded may also be referred to as side-loaded or laterally loaded since the shaft of the EMD is loaded into the collet 176 through a longitudinal side of the collet body (that is the side of the collet body extending from a proximal end to the distal end of the collet body). Radially loading, side loading or laterally loading is in contrast to axially loading in which a shaft portion is loaded into the internal pathway by first inserting a free end of the shaft into a proximal or distal opening in the collet's internal pathway. In one embodiment the collet 176 includes at least two members that move relative to each other to releasably fix the shaft portion of the EMD to at least one of the two members. In one embodiment the two members operating together provide a mechanical advantage that increases the torque and /or force that may be transmitted from the collet body to the shaft of the EMD without the shaft of the EMD moving relative to the collet body. The pinch force on the EMD using a collet can be greater than the force required to actuate the pinch. When the shaft of the EMD is pinched it is fixed such that there is relative movement of the collet and EMD during acceptable operation parameters of an EMD procedure.

Although the present disclosure has been described with reference to example embodiments, workers skilled in the art will recognize that changes may be made in form and detail without departing from the spirit and scope of the defined subject matter. For example, although different example embodiments may have been described as including one or more features providing one or more benefits, it is contemplated that the described features may be interchanged with one another or alternatively be combined with one another in the described example embodiments or in other alternative embodiments. The present disclosure described is manifestly intended to be as broad as possible. For example, unless specifically otherwise noted, the definitions reciting a single particular element also encompass a plurality of such particular elements.

## Claims

1. An EMD drive system comprising:
a robotic drive (24) having a robotic drive longitudinal axis (78);
a device module (32d) (32c) (32b) (32a) movable along the robotic drive longitudinal axis (78), the device module (32d) (32c) (32b) (32a) including a motor (102) having a motor shaft (108) being substantially parallel to the robotic drive longitudinal axis (78); and
a drive train (104) coupling the motor shaft (108) to a driven member (90) configured to rotate an elongated medical device about an EMD longitudinal axis.

2. The EMD drive system of claim 1, the motor (102) has a first length along a motor longitudinal axis (114) less than a second longitudinal length of the device module (32d) (32c) (32b) (32a) taken along a longitudinal axis parallel to the robotic drive longitudinal axis (78).

3. The EMD drive system of claim 1, wherein the drive train (104) includes a drive gear (88) rotating about an axis (172) perpendicular to a motor longitudinal axis (114) of the motor shaft (108), the drive gear (88) removably engaged with the driven member (90).

4. The EMD drive system of claim 1, further including a stage member (82) extending from the robotic drive (24), the stage member (82) moving the device module (32d) (32c) (32b) (32a) along the robotic drive (24) longitudinal axis (78), the device module extending solely from a first side (84) of the stage member (82).

5. The EMD drive system of claim 1, wherein the drive train (104) includes a worm gear (122) and a worm wheel (124).

6. The EMD drive system of claim 5, wherein the drive train (104) includes an intermediate shaft (116) parallel to the motor shaft (108), the worm gear (122) being rotated by the intermediate shaft (116) and the worm gear (122) being intermediate a proximal end (134) of the motor (102) and a distal end (170) of the motor (102).

7. The EMD drive system of claim 3, further including a collet (176) removably received within the device module (32d) (32c) (32b) (32a) between a collet in-use position and a collet external position, the collet (176) being manually adjustable in the collet in-use position between a first fixed position affixing an EMD thereto and a second unfixed position in which the EMD is not affixed thereto.

8. The EMD drive system of claim 7, wherein the drive train (104) rotates the collet (176) about a collet longitudinal axis, the drive train (104) rotating the EMD when the EMD is fixed to the collet (176).

9. The EMD drive system of claim 8, wherein the device module (32d) (32c) (32b) (32a) includes a drive module (60) (62) (64) (66) and a cassette (68) (70) (72) (74) removably coupled to the drive module, the drive module including a housing (80) supporting the motor (102) and the drive gear (88), the cassette (68) (70) (72) (74) including the driven member (90) and removably receiving the collet (176) and the EMD.

10. The EMD drive system of claim 9, wherein the collet (176) includes a first portion (178) operatively connected to the drive train (104) and a second portion (180) movable by a user with respect to the first portion (178) to fix and unfix the EMD to the collet, wherein the second portion is proximal to the first portion when the collet is in an in-use position.

11. The EMD drive system of claim 7, wherein the drive train (104) prevents back drive in response to a force applied to the collet (176) to adjust the collet (176) between the first fixed position and the second unfixed position.

12. The EMD drive system of claim 7, wherein the drive train (104) rotates the collet (176) about a longitudinal axis of the collet (176), the drive train (104) rotating the EMD when the EMD is fixed to the collet (176).

13. The EMD of claim 7, wherein the collet (176) includes a gear (174) operationally engaged with the driven member (90) and a proximal portion (180) being manipulated by a user when the collet (176) is in the collet in-use position.

14. An EMD drive system comprising:
a robotic drive (24) having a robotic drive longitudinal axis (78), the robotic drive (24) having a housing including a bottom surface (92) being closest to a patient in a robotic drive in-use position;
a drive module (60) (62) (64) (66) movable along the robotic drive longitudinal axis (78), the drive module extending from the robotic drive (24) with a bracket (82) defining a drive plane extending along the robotic drive longitudinal axis (78) and extending perpendicular to the bottom surface (92), the drive module extending solely from one side of the drive plane, the drive module including a motor (102) and a drive train (104) operationally coupling the motor (102) to an EMD within the drive module; and
a sterile barrier (94) removably attached to the bottom surface (92) of the robotic drive (24) on a second side of the drive plane opposite the one side of the drive plane.

15. The EMD drive system of claim 14, wherein the motor (102) comprises a drive shaft (108) that has a longitudinal axis that is parallel with the drive plane.

16. The EMD drive system of claim 15, further including a cassette (68) (70) (72) (74) removably attached to the drive module, and a collet (176) being removably received within the cassette (68) (70) (72) (74) between a collet external position and a collet in-use position, a portion of the collet (176) being operatively connected to the drive train (104).

17. The EMD drive system of claim 16, wherein the drive train (104) includes a worm gear (122) preventing back drive of the drive train (104) when the collet (176) is manually adjustable in the collet in-use position between a first fixed position affixing the EMD to the collet and a second unfixed position in which the EMD is not affixed to the collet.

18. An EMD drive system comprising:
a robotic drive (24) having a robotic drive longitudinal axis (78);
a device module (32d) (32c) (32b) (32a) movable along the robotic drive longitudinal axis (78), the device module (32d) (32c) (32b) (32a) including a motor (102) having a motor shaft (108); and
a drive train (104) coupling the motor shaft (108) to a driven member (90) configured to rotate an elongated medical device about an EMD longitudinal axis (184), the drive train (104) including a worm gear (122) preventing back drive of the drive train (104) when a force is applied to the drive train (104) from an EMD.

19. The EMD drive system of claim 18, wherein a motor longitudinal axis (114) of the motor shaft (108) is parallel to the robotic drive longitudinal axis (78).

20. The EMD drive system of claim 19, wherein the drive train (104) includes an intermediate shaft (116) extending parallel to the motor shaft (108), the worm gear (122) being positioned on the intermediate shaft (116), the driven member (90) rotating about an axis (172) spaced from and perpendicular to the motor longitudinal axis (114) and a longitudinal axis (118) of the intermediate shaft (116), and wherein the worm gear (122) is positioned on the intermediate shaft (116).
